Europäisches Patentamt

European Patent Office

Office européen des brevets

(11) Publication number: 0 247 560
A1

## EUROPEAN PATENT APPLICATION

(21) Application number: 87107588.3

(22) Date of filing: 25.05.87

(51) Int. Cl.⁴ A61B 5/04

(30) Priority: 28.05.86 JP 80853/86 U

(43) Date of publication of application:
02.12.87 Bulletin 87/49

(84) Designated Contracting States:
DE FR GB IT

(71) Applicant: FUKUDA DENSHI CO., LTD.
39-4, Hongo 3-chome Bunkyo-ku
Tokyo 113(JP)

Applicant: SANSHIN KASEI CO., LTD.
23-4, Higashigotanda 5-chome Shinagawa-ku
Tokyo 113(JP)

(72) Inventor: Shimuzu, Chuji
No. 2-2-3 Narishinodai,
Funabashi-shi Chiba-ken,(JP)
Inventor: Miura. Hiroshi
23-4, Higashigotanda 5-chome,
Shinagawa-ku Tokyo 113,(JP)

(74) Representative: Patentanwälte TER MEER -
MÜLLER - STEINMEISTER
Mauerkircherstrasse 45
D-8000 München 80(DE)

(54) Lead securement sheet for electrocardiographic electrode.

㊿ A lead securement sheet (4) for an electrocardiographic electrode (1) is disclosed, which comprises an urging section (5) for urging an electrocardiographic electrode lead (2), an attaching/separating section (7) to be manipulated to attach and separate the sheet and an adhesive section (6) to be bonded to the skin of a man. The urging and attaching/separating sections are not sticky, while the adhesive section is sticky.

FIG. 2

## LEAD SECUREMENT SHEET FOR ELECTROCARDIOGRAPHIC ELECTRODE

This invention relates to a lead securement sheet for an electrocardiographic electrode, which holds urged to a man a lead of an electrocardiographic electrode held in close contact with the man's skin for deriving minute current generated in the man, thus reinforcing the stability of close contact of the electrocardiographic current derivation electrode to the man.

As is well known in the art, electric current is induced in a man by the activities of the heart, brain, muscles, etc.

Particularly, electricity generated in the heart is recorded by an externally provided electrocardiograph as minute current induced at the man's skin for effecting diagnosis of the heat.

To this end, an input section of the electrocardiograph is electrically coupled to the man. This means that it is necessary to have electrocardiographic current derivation electrodes in close contact with the man's skin.

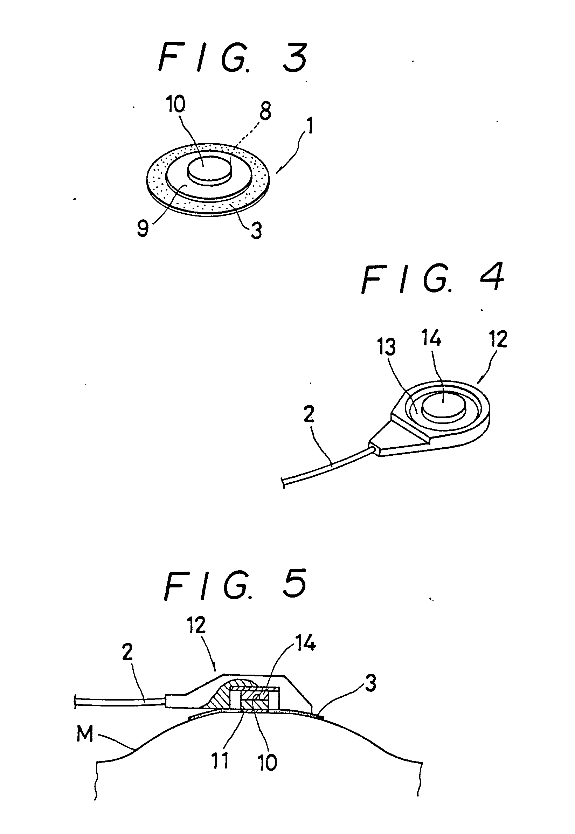

A prior art electrocardiographic electrode to be held in close contact with the man's skin will now be described with reference to Figs. 3 to 5. Fig. 3 is a perspective view of the electrocardiographic electrode 1. The electrode 1 has a substantially circular sticky piece 3. The sticky piece 3 is a doughnut-like woven cloth having a central opening 8. Its back side has stickiness to be held in close contact with the skin M of a man, as shown in Fig. 5.

To the top side of the sticky piece 3 is bonded an electrode holder 9 made of a hard synthetic resin such as to close the opening 8. The electrode holder 9 has a magnetic lead coupler 10 projecting from the top side.

To the underside of the lead coupler 10 is secured an electrode 11, as shown in Fig. 5, for deriving minute current from the heart in direct contact with the skin M of the man.

Fig. 4 shows the back side of a lead connector 12 for leading the minute voltage in the heart derived from the electrode 11 through the electrocardiographic lead 2 to an electrocardiograph provided in a room.

The head connector 12 is made of a hard resin and has substantially the same size as the electrocardiographic current derivation electrode 1. The lead connector 21 has a recess 13. An electromagnetic electrode coupler 14 is received in the recess 13 and secured to the lead connector 12.

To record an electrocardiogram using the electrocardiographic electrode 1 having the above construction, the sticky piece 3 of the electrocardiographic electrode 1 is applied to the skin M of a man, as shown in Fig. 5. Then, the electromagnetic electrode coupler 14 of the lead connector 12 is bonded to the lead coupler 10 of the electrocardiographic electrode 1, thus coupling the lead connector 12 to the electrocardiographic electrode 1. In this state, minute voltage from the heart derived from the electrode 11 is supplied through the electrocardiographic lead 2 to an electrocardiograph (not shown).

Generally, in a heavy disease patient in a hospital, the electrocardiographic electrode 1 is held in close contact with the patient's skin, and minute current derived from the patient's skin is led to an electrocardiograph installed in a separate heavy disease patient monitor room to be monitored by a monitor.

However, when the lead connector 12 coupled to the electrocardiographic electrode 1 held in close contact with the patient's skin is liable to be detached from the electrocardiographic electrode 1 due to an unconsious motion of the patient such as a turn-round in sleep. In such a case, noise is generated so that an accurate electrocardiogram can not be obtained.

Further, the patient may unconsiously pull the electrocardiographic electrode 2 of the lead connector 12. When the lead 2 is pulled, it is detached from the electrocardiographic electrode 1 in an extreme case. When the lead connector 12 is detached, an alarm device provided in the heavy disease patient monitor room is operated, causing the monitor to rush into the patient room to see the patient's condition.

The alarm device is designed to generate an alarm when there arises an energent condition of the patient, e.g., stoppage of the heartbeats while the patient's electrocardiogram is being recorded with the electrocardiographic electrode 1 held in close contact with the patient's skin. Despite this intend, the alarm device is operated every time the lead connector 12 or electrocardiographic electrode 1 is detached from the patient's skin in spite of the fact that the patient's heart is normal. Whenever the alarm device is operated even for misalarming, the monitor rashes from the heavy disease patient monitor room to the patient room. This extremely increases the fatigue of the monitor due to misalarming.

To cope with the above problem, it has been in practice to secure the lead to the skin M of the patient with an adhesive tape.

In this method, the adhesive that is coated on the entire surface of the tape is attached to the lead. After use, therefore, the lead is wiped with alcohol for maintenance. In this case, in long use the lead is deteriorated and becomes hard so that it can no longer be used.

Further, since the entire surface of the tape is sticky, with a movement of the patient the lead 2 of the lead connector 12 that is secured by the tape to the skin M is pulled. Consequently, the lead connector 12 is liable to be deviated from the electrocardiographic electrode 1, resulting in generation of noise.

Further, since the tape is sticky over the entire surface, it can be difficultly separated from the skin.

An object of the invention is to solve problems arising form the fact that the prior art tape for securing the electrocardiographic lead 2 to the skin is sticky over the entire surface.

To attain the above object of the invention, there is provided a lead securement sheet for an electrocardiographic electrode, which comprises an urging section for urging an electrocardiographic electrode lead, an attaching/separating section to be manipulated to attach and separate the sheet and an adhesive section to be bonded to the skin of a man, the urging and attaching/separating sections being not sticky, said adhesive section being sticky.

Since the electrocardiographic electrode lead is urged by the non-sticky urging section of the sheet, it can be moved between the sheet and skin to follow the movement of the man. Therefore, the lead connector is never pulled with the movement of the man, and the lead connector thus can be stably held without being detached from the electrocardiographic electrode.

Further, since the urging section is not sticky, no adhesive is attached to the lead when the lead is urged.

When removing the device with the electrocardiographic lead, the non-sticky attaching/separating section is pulled by pinching it with fingers, so that the electrocardiographic electrode lead securement sheet can be readily separated from the skin.

Fig. 1 is a view for explaining an embodiment of the invention;

Fig. 2 is a view showing an electrocardiographic electrode securement sheet according to the invention;

Fig. 3 is a perspective view showing a prior art electrocardiographic electrode;

Fig. 4 is a perspective view showing the back side of a lead connector; and

Fig. 5 is a view showing an electrocardiographic electrode and a lead connector in use.

Now, the constitution and operation of the lead securement sheet according to the invention will be described in detail in conjunction with an embodiment thereof with reference to the drawings.

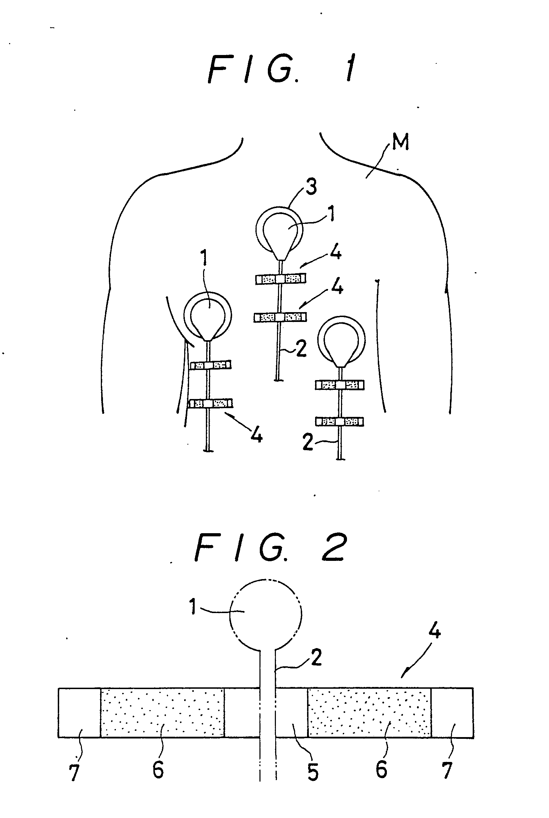

Fig. 1 is a view showing an embodiment of the invention. Reference symbol M designates the skin of a man. Reference numeral 1 designates electrocardiographic electrodes, 2 electrocardiographic electrode leads, and 3 electrocardiographic electrode securerment sheets for bonding the electrocardiographic electrodes 1 to the skin of the man.

Reference numeral 4 designates lead securement sheets for the electrocardiographic electrodes. The lead securement sheet 4 serves to secure the electrocardiographic electrode lead 2 to the skin M of the man, thus further stabilizing the securement of the electrocardiographic electrode 1.

Fig. 2 is a view showing the lead securement sheet for an electrocardiographic electrode. The Figure shows the back side of the sheet for the sake of the description.

Referring to the Figure, referring to the Figure, reference numeral 5 designates an urging section for urging the electrocardiographic electrode lead 2 against the skim M of the man to reinforce the stabilization of the securement of the electrocardiographic electrode 1.

The urging section 5 is not sticky, so that no adhesive sticks to the lead.

Reference numeral 6 designates adhesive sections which are made of an adhesive and sticky. These adhesive sections of the sheet are applied to the skin M of the man.

Since the adhesive sections 6 are on the opposite sides of the urging section 5, even if they are applied to the skin M, the electrocardiographic electrode lead 2 is not secured to the skin but is held slidably with respect thereto by the urging section 5.

Therefore, even if the man with the electrocardiographic lead 2 moves the body, the lead 2 slides in the urging section 5 and does not pull but follow the movement of the electrocardiographic electrode 1. Therefore, the electrocardiographic electrode 1 secured to the skin M of the man is held stable at all time irrespective of the movement of the man's body.

Reference numeral 7 designates attaching/separating sections. These sections are non-sticky, so that these sections can be readily separated by pulling these sections by clipping them with fingers. This facilitates the operation of removing the device with the electrocardiographic electrode lead 2.

As has been described in the foregoing, with the electrocardiographic elelctrode lead securement sheet according to the invention, while the adhesive sections are sticky, the urging section for urging the electrocardiographic electrode lead to the man's skin isnot sticky, so that no adhesive will stick to the electrocardiographic electrode lead. Thus, the electrocardiographic electrode lead is neither stained nor deteriorated, so that it can be used for long time.

Further, the electrocardiographic electrode lead is not applied to the skin but is held slidably with respect thereto in the urging section. Therefore, it can follow the movement of the electrocardiographic electrode moved with the man's body. That is, the electrocardiographic electrode and electrocardiographic electrode lead are flexibly secured to and held by the man's skin and is thus highly stable with respect to the movement of the man's body.

Further, since the attaching/separating sections are non-sticky, when removing the device with the electrocardiographic electrode and electrocardiographic electrode lead, the lead securement sheet for an electrocardiographic electrode can be readily separated by pulling the attaching/separating sections with fingers.

## Claims

A lead securement sheet (4) for an electrocardiographic electrode (1) comprising an urging section (5) for urging an electrocardiographic electrode lead (2), an attaching/separating section (7) to be manipulated to attach and separate said sheet and an adjesive section (6) to be bonded to the skin of a man, said urging and attaching/separating sections being not sticky, said adhesive section being sticky.

# F I G.  1

M

3
1
4
4
2
1
2
4

# F I G.  2

1
2
4
7    6    5    6    7

# F I G. 3

# F I G. 4

# F I G. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 568 766 (G. ASCHER et al.) * page 4, lines 14-18; figure 1 * | 1 | A 61 B 5/04 |
| A | US-A-4 332 257 (G.E. AYER) * column 3, lines 39-51; figure 1 * | 1 | |
| A | US-A-4 331 153 (J.W. MEALY) * column 4, lines 6-17; figure 5 * | 1 | |
| A | DE-A-2 802 547 (SIEMENS AG) * page 6, lines 6-14; figure 1 * | 1 | |

----

|  | TECHNICAL FIELDS SEARCHED (Int. Cl.4) |
|---|---|
|  | A 61 B 5/00<br>A 61 N 1/00 |

The present search report has been drawn up for all claims

| Place of search | Date of completion of the search | Examiner |
|---|---|---|
| BERLIN | 03-08-1987 | WEIHS J.A. |